# FASCICULE DE BREVET EUROPEEN

(11) **EP 3 658 071 B1**
(45) Date de publication et mention de la délivrance du brevet: **29.06.2022**
(21) Numéro de dépôt: 18742802.4
(22) Date de dépôt: 20.07.2018
(51) Int. Cl.: B08B 1/00

(54) **LINGETTES D'HYGIÈNE DENTAIRE COMPRENANT DES MICROCAPSULES**
REINIGUNGSTÜCHER FÜR DIE ZAHNHYGIENE, DIE MIKROKAPSELN ENTHALTEN
DENTAL HYGIENE WIPES COMPRISING MICROCAPSULES

(30) Priorité: 24.07.2017 EP 17182835
(43) Date de publication de la demande: 03.06.2020
(73) Titulaire: Bochenek, Stéphane, 91330 Yerres (FR)
(72) Inventeur: GOFFINET, Pierre, 91190 Gif-Sur-Yvette (FR); BOCHENEK, Stéphane, 91330 YERRES (FR)
(86) Numéro de dépôt international: PCT/EP2018/069844
(87) Numéro de publication internationale: WO 2019/020535

(56) Documents cités:
- WO-A2-03/015656
- JP-U- H0 529 516
- US-A- 3 902 509
- US-A1- 2003 120 180

## Description

La présente invention est relative à des produits d'hygiène dentaire à usage unique, de type lingette.

Il est généralement reconnu qu'un nettoyage des dents après chaque repas est non seulement souhaitable pour une bonne hygiène bucco-dentaire, mais procure en outre une sensation de propreté, de fraîcheur, et de bien-être buccal appréciée par la plupart des personnes.

Les moyens usuels de nettoyage des dents, tels que la brosse à dents et le dentifrice, les bains de bouche, les jets dentaires, etc. ne sont pas adaptés à une utilisation en cours de journée en dehors du domicile, car outre leur caractère encombrant, ils nécessitent d'avoir accès à un point d'eau, ce qui n'est pas toujours le cas.

De nombreux articles d'hygiène dentaire destinés aux personnes désireuses de se nettoyer les dents en tout lieu et à tout moment de la journée ont été décrits dans l'art antérieur. On citera notamment des dispositifs jetables de type lingette, comportant un support textile imprégné d'une composition nettoyante. De tels dispositifs peuvent se présenter sous des formes variées. Fréquemment, ils sont en forme de doigt de gant, ou de feuille repliable pour s'adapter au doigt de l'utilisateur. De tels dispositifs sont par exemple décrits dans les Brevets US 3 902 509, US 4 335 731, EP 1 267 663, ou FR 3 022 134.

Il a été proposé (Brevet US 6 898 819 ; Brevet US 6 721 987 ; Brevet US 3 902 509) d'encapsuler certains des composants de la composition nettoyante, notamment les arômes, pour les protéger contre les altérations de leurs propriétés chimiques et physiques. La matière d'encapsulation utilisée est soluble dans l'eau et libère le contenu des capsules lors du contact avec l'humidité de la cavité buccale. Ces capsules étant d'un seul type, leur contenu est libéré simultanément.

US 2003/120180 A1 divulgue une lingette de nettoyage dentaire comprenant un support textile non tissé dont au moins une partie des constituants sont encapsulés dans des microcapsules déposées à la surface dudit support, les microcapsules étant à rupture mécanique dont le contenu est libéré par rupture de la paroi.

La présente invention a pour but de fournir des lingettes dentaires permettant à la fois (a) d'obtenir un effet immédiat de propreté et de fraîcheur, et (b) d'obtenir un effet différé de fraîcheur persistant plus longtemps après l'application que celui des lingettes dentaires de l'art antérieur.

La présente invention a ainsi pour objet une lingette de nettoyage dentaire comprenant un support textile non-tissé imprégné d'une composition d'hygiène dentaire appropriée dont au moins une partie des constituants (tels que des agents aromatisants, des agents antiseptiques, des huiles essentielles ou extraits végétaux, et optionnellement des agents anti-plaque, des agents anti-caries et des agents blanchissants) sont encapsulés dans des microcapsules déposées à la surface dudit support, ladite lingette étant caractérisée en ce que 2 types différents de microcapsules sont associés :
(a) des microcapsules à rupture mécanique dont le contenu est immédiatement libéré par rupture de la paroi, et
(b) des microcapsules à libération différée et contrôlée dont le contenu est libéré par perméation à travers la paroi.

a) Le premier type de microcapsules (dénommées également ci-après « à rupture mécanique ») peut libérer son contenu par rupture de la paroi de la microcapsule sous l'effet de la pression exercée sur les dents par l'utilisateur lors de l'application de la lingette.
b) Le second type de microcapsules (dénommées également ci-après « à diffusion contrôlée ») peut libérer son contenu par perméation contrôlée de celui-ci à travers la paroi de la microcapsule.

La combinaison de ces deux types de microcapsules a pour principal avantage d'apporter à la fois une efficacité immédiate à l'usage par libération instantanée des actifs contenus dans les microcapsules à rupture mécanique et de prolonger cette efficacité dans le temps par perméation des actifs à travers les parois des microcapsules à diffusion contrôlée déposées sur les dents et les gencives par le frottement de la lingette.
(a) Les microcapsules à rupture mécanique ont une paroi qui doit être insoluble en solution aqueuse et suffisamment résistante pour ne pas céder aux contraintes mécaniques intervenant lors de la fabrication, du transport et du stockage du produit. En revanche, elles doivent pouvoir se rompre et libérer leur contenu sous la pression exercée par l'utilisateur final lors de l'application de la lingette. Ainsi, 80% au moins de ces microcapsules doivent pouvoir résister à une pression égale à 20 g/cm², et environ 90% de ces microcapsules doivent se rompre à une pression égale à 500 g/cm².
(b) Les microcapsules à diffusion contrôlée sont insolubles en milieu aqueux et ne doivent pas se rompre sous la pression exercée lors de l'application de la lingette. Elles doivent donc pouvoir résister à des pressions de l'ordre de 2 000 g/cm².

La nécessité du caractère insoluble ou très faiblement soluble dans l'eau des parois des microcapsules qu'elles soient à rupture mécanique ou à diffusion contrôlée, tient à la nature des compositions d'hygiène dentaire revendiquées qui contiennent généralement une proportion notable d'eau ; si cette condition ne se trouvait pas respectée, la composition solubiliserait elle-même le matériau d'encapsulation.

En outre, dans le cas des microcapsules à diffusion contrôlée, l'hydrosolubilisation rapide de la membrane d'encapsulation au contact de la salive conduirait nécessairement à une libération instantanée de son contenu, allant à l'encontre de l'effet de libération différée recherché.

La proportion des microcapsules à rupture mécanique et des microcapsules à diffusion contrôlée peut varier en pourcentage relatif dans un rapport de 95/5 à 5/95.

La paroi des microcapsules est constituée à partir d'un matériau substantiellement insoluble dans l'eau ou rendu non-hydrosoluble par un agent précipitant ou réticulant. Elle est préférentiellement constituée de résine mélamine à bas taux de formol résiduel, de carboxyméthylcellulose (CMC) réticulée, de gélatine réticulée de peau de porc, de boeuf ou de collagène marin, de polyuréthane, de polyamide, de polyacrylate, de silicone (diméthicone) ou de silicone modifiée. Elle peut être également constituée de dérivés de cellulose, de polysaccharides, de chitosane, de polyols, d'alcool polyvinylique, de polyvinylpyrolidone, de PLGA-PLA (Poly (D,L-acide lactique-co-glycolique)- Poly (D,L-acide lactique)) ou de polycaprolactone, rendus insolubles dans l'eau par réticulation ou précipitation.

Les microcapsules à diffusion contrôlée sont de préférence obtenues à partir de gélatine réticulée, de polyamide, ou de silicones. Leur paroi doit permettre une diffusion de leur contenu sur une durée contrôlable en fonction du diamètre moyen et du nombre de micropores dans leur structure ; cette durée peut varier de quelques minutes à plusieurs jours. La diffusion peut également être contrôlée par l'épaisseur de la paroi ; l'épaisseur et la porosité de la paroi des microcapsules sont déterminée pour permettre une étanchéité suffisante en conditions normales de stockage, mais une libération progressive de leur contenu pendant une durée variant de quelques minutes à quelques heures, sous l'effet de la température buccale et/ou de la salive.

Ces microcapsules peuvent être préparées par des procédés connus en eux-mêmes de l'homme du métier. Dans la plupart des cas, selon les conditions choisies pour la mise en œuvre du procédé, on peut obtenir des microcapsules à rupture mécanique ou des microcapsules à diffusion contrôlée.

Un agent réticulant permettant d'insolubiliser le polymère est par exemple un dichlorure d'acide (dans le cas de la gélatine) ou du borax (dans le cas de l'alcool polyvinylique), qui va ponter le polymère à l'interface et former ainsi une membrane insoluble en périphérie des gouttelettes d'actif à encapsuler.

Un précipitant insolubilisant peut-être par exemple un sel de calcium ou de magnésium (cas de la carboxyméthyl cellulose).

En considération de la nature de l'invention, on veille naturellement à ce que d'éventuelles traces résiduelles d'agents réticulants ou précipitants ou de leurs dérivés réactionnels ne constituent pas un risque toxicologique par ingestion ou contact avec les muqueuses.

À titre d'exemples non limitatifs de préparation des microcapsules on mentionnera ci-après l'encapsulation par coacervation complexe, l'encapsulation par polymérisation in situ et l'encapsulation par polymérisation interfaciale
a) Encapsulation par coacervation complexe
   Les microcapsules sont obtenues par formation d'un complexe de gélatine et d'un polymère anionique (CMC, polyphosphate, alginate, gomme arabique etc..) sous l'influence du pH.

La présence d'un polymère anionique dit colloïde protecteur est obligatoire pour obtenir des capsules isolées ou en grappes.

Le procédé comprend 5 étapes :
Émulsion
Coacervation
Compactage
Réticulation
Remontée en température et ajustement du pH

On obtient ainsi des capsules souples et transparentes, de diamètre pouvant varier de 1 µm à 500 µm.

Selon leur degré de réticulation et leur taille, ces capsules peuvent être étanches et se rompre par application d'une pression déterminée (capsules à rupture mécanique) ou perméables, permettant un relargage contrôlé avec une vitesse de relargage qui peut être ajustée en fonction des besoins.

Les capsules de dimensions plus importantes ont tendance à générer une plus grande porosité des parois et créent ainsi des conditions plus favorables à une perméation contrôlée des actifs.

### b) Encapsulation par polymérisation in situ de résine

Ce procédé permet la libération de l'actif par bris de l'enveloppe avec une fragilité réglable de cette dernière (microcapsuies à rupture mécanique).

La réalisation de microcapsules par polymérisation de l'enveloppe est obtenue en phase aqueuse à partir d'une solution de polymère qui devient insoluble et précipite autour des gouttes d'un composé huileux ou faiblement soluble dans i'eau sous l'effet d'une variation de pH, de température ou par ajout d'un agent réticulant.

L'opération se fait en 2 étapes modulées par le pH et la température :
Émulsion et polymérisation : c'est le polymère en formation qui stabilise l'émulsion
Réticulation

Elle nécessite également la présence d'un polymère anionique dit colloïde protecteur.

Les résines mélamine et urée formol sont les principales utilisées.

La libération des principes actifs est obtenue par bris mécanique de l'enveloppe ; la fragilité de la paroi de la microcapsule est réglable en fonction du temps de réticulation.

Ces microcapsules dures et opaques présentent une très bonne étanchéité et très bonne résistance chimique, avec un diamètre moyen compris entre 1 µm et 200 µm.

### c) Encapsulation par polymérisation in situ de résine silicone

Le principe est proche du précédent, utilisant des monomères de silicone ou de silicone fonctionnalisée.

On obtient des capsules souples et transparentes, de diamètre compris entre 1 µm et 500 µm.

Selon le degré de réticulation et leur taille, ces capsules peuvent libérer leur contenu par rupture de l'enveloppe, ou permettre un relargage contrôlé.

### d) Encapsulation par polymérisation interfaciale

Le principe est la formation d'un polymère par réaction entre deux monomères ou pré polymères, l'un des deux produits étant dans la phase aqueuse et l'autre dans la phase huileuse.

Le procédé se déroule en 2 étapes modulées par le pH et la température :
Émulsion et polymérisation : c'est le polymère en formation qui stabilise l'émulsion ;
Réticulation.

Les principaux polymères utilisés sont les polyuréthanes (diisocyanate + diamine) et les polyamides (chlorure de diacide + diamine).

Ce procédé permet d'obtenir des microcapsules à rupture mécanique, mais également, si souhaité, des microcapsules à diffusion contrôlée si l'épaisseur de paroi est rendue plus faible, notamment en réduisant le temps de réticulation.

Les lingettes dentaires conformes à l'invention peuvent se présenter sous différentes formes, par exemple sous forme de feuillets plats. Toutefois, elles se présentent de préférence sous la forme d'un doigt de gant.

Le support non-tissé utilisé dans ces lingettes dentaires est de préférence constitué de cellulose, de cellulose modifiée chimiquement ou d'une matière absorbante dérivée de cellulose.

Notamment lorsque les lingettes sont sous forme de doigt de gant, le support non-tissé est soudé ou collé sur une couche de film imperméable, positionnée sur la face interne du doigt de gant. Ce film imperméable forme une barrière permettant de protéger le doigt de l'utilisateur de la salive, ainsi que d'éviter la diffusion à l'intérieur du doigt de gant de la composition imprégnant le support non tissé. Ce film barrière peut être constitué, à titre d'exemples non limitatifs, de PVC, de polyéthylène, de polypropylène, de polyamide, de films complexes co-extrudés, etc....

Le support non-tissé présente une épaisseur comprise entre 20 et 1500 µm, cette épaisseur étant de préférence au moins égale à 2 fois le diamètre moyen des microcapsules déposées à sa surface. Lorsque le processus de fabrication et de stockage du non tissé implique une opération de bobinage, la résistance de la microcapsule associée à l'élasticité du non tissé doit permettre d'absorber une pression de bobinage jusqu'à 20 g/cm2 sans rupture prématurée des microcapsules.

Le diamètre moyen des microcapsules renfermant les actifs utiles pour l'invention est compris entre 1 et 500 µm, de préférence entre 1 et 50 µm, de préférence encore entre 2 et 20 µm.

La quantité de microcapsules déposée sur le non tissé est de l'ordre de 5 à 50 g/m2 de surface du support non tissé.

La composition d'hygiène dentaire imprégnant le non-tissé peut contenir des constituants divers choisis parmi ceux habituellement utilisés dans les compositions d'hygiène bucco-dentaire ; à titre d'exemples non-limitatifs, il peut s'agir par exemple d'agents tensioactifs, d'agents de polissage, d'agents blanchissants, d'agents anti-plaque, d'agents anti-caries, d'agents antiseptiques, d'agents aromatisants, etc. Généralement, une partie des composants est incluse dans les microcapsules, et l'autre partie imprègne directement le support non-tissé. Les composants inclus dans les microcapsules seront généralement des agents aromatisants et/ou antiseptiques, par exemple des essences végétales, et le cas échéant des agents anti-plaque, et/ou des agents anti-caries.

Ces microcapsules sont fixées à la surface du non tissé par pulvérisation, foulardage (trempage), couchage ou enduction.

En vue d'améliorer leur stabilité et leurs propriétés d'adhésion au non tissé, ces microcapsules peuvent être associées à des adjuvants tels que : liants, émulsifiants, conservateurs jusqu'à 30 % en masse par rapport à celle des microcapsules.
Émulsionnants : par ex. du polysorbate 80 (ester de sorbitan éthoxylé), de la lécithine hydrogénée, du monostéarate de glycérol, etc....
Liants (0,5 à 1,5 % en masse) : tous agents dont les propriétés d'épaississant et de stabilisant permettent d'augmenter le pouvoir adhésif des microcapsules sur le non tissé, comme : la carboxyméthylcellulose, la gomme cellulosique, la gomme xanthane, etc....
Conservateurs : par ex. les dérivés d'isothiazolinone, le benzoate de sodium, le sorbate de potassium, l'EDTA, l'acide déhydroacétique, etc....

La présente invention sera mieux comprise à l'aide du complément de description qui va suivre, qui se réfère à des exemples non-limitatifs illustrant la préparation de lingettes dentaires conformes à l'invention.

### Exemple 1 : Nettoyant dentaire désinfectant 1

| Composant | % dans microcapsule | % dans formule |
|---|---|---|
| Eau purifiée | | 71,20 |
| Glycérine | | 5,00 |
| Xylitol | | 0,50 |
| Sorbitol | | 2,00 |
| Cocoyl glucoside | | 0,20 |
| Benzoate de sodium | | 0,30 |
| Citrate de sodium | | 0,80 |
| Microcapsules silicone à diffusion contrôlée diamètre moyen 5 µm contenant ; | | 7,00 |
| Huile essentielle de *Melaleuca Altemifolia* | *10,00* | |
| Huile essentielle de Menthe poivrée | *30,00* | |
| Huile essentielle de Citron | *60,00* | |
| Microcapsules gélatine réticulée diamètre moyen 15 µm à rupture mécanique contenant : | | 13,00 |
| Huile essentielle de Citron | *70,00* | |
| Huile essentielle de Menthe Poivrée | *30,00* | |
| TOTAL | | 100,00 |

Les microcapsules en silicone à diffusion contrôlée sont préparées par polymérisation in situ.

Les microcapsules en gélatine à rupture mécanique sont préparées par coacervation complexe en présence d'alginate.

Une lingette en forme de doigtier, constituée d'une couche de cellulose d'épaisseur 50 µm, doublée d'un film imperméable de 20 µm d'épaisseur a été imprégnée par foulardage avec la composition indiquée dans le tableau ci-dessus, cette composition étant préparée par simple mélange à froid ; les deux types de microcapsules, se présentant tous deux sous la forme d'une épaisse suspension aqueuse à la concentration d'environ 35% de capsules sèches, y sont ajoutées en fin de mélange sous faible agitation.

### Exemple 2 : Nettoyant dentaire 2

| Composant | % dans microcapsule | % dans formule |
|---|---|---|
| Eau purifiée | | 81,90 |
| Glycérine | | 3,00 |
| Xylitol | | 0,80 |
| Bicarbonate de sodium | | 2,00 |
| Benzoate de sodium | | 0,30 |
| Sorbate de potassium | | 0,20 |
| Carboxyméthyl cellulose | | 1,20 |
| Polysorbate 80 | | 0,60 |
| Microcapsules silicones à diffusion contrôlée diamètre moyen 5 µ contenant : | | 7,00 |
| Huile essentielle d'Eucalyptus Globulus | *30,00* | |
| Huile essentielle de Citron | *70,00* | |
| Microcapsules gélatine réticulée de poisson diamètre moyen 15 µ à rupture mécanique contenant : | | 3,00 |
| Huile essentielle d'Eucalyptus Globulus | *30,00* | |
| Huile essentielle de Citron | *70,00* | |
| TOTAL | | 100,00 |

Les microcapsules en silicone à diffusion contrôlée sont préparées par polymérisation in situ.

Les microcapsules en gélatine à rupture mécanique sont préparées par coacervation complexe en présence de carboxyméthyl cellulose.

Une lingette en forme de doigtier, constituée d'une couche de cellulose d'épaisseur 50 µm, doublée d'un film imperméable de 20 µm d'épaisseur a été imprégnée par pulvérisation de la partie cellulosique avec la composition indiquée dans le tableau ci-dessus, la composition hors microcapsules étant préparée par simple mélange à la température de 45°C ; après refroidissement à 30°C maximum, les microcapsules y sont ajoutées sous faible agitation. Les microcapsules silicone à diffusion contrôlée sont intégrées sous forme d'une épaisse suspension aqueuse à la concentration de 35% de capsules sèches et les microcapsules de gélatine de poisson à rupture mécanique se présentent sous forme de capsules sèches et sont introduites en fin de procédé de mélange.

### Exemple 3 : Nettoyant dentaire désinfectant 3

| Composant | % dans microcapsule | % dans formule |
|---|---|---|
| Eau purifiée | | 82,92 |
| Glycérine | | 5,00 |
| Acésulfame de potassium | | 0,05 |
| Fluorure de sodium | | 0,03 |
| Benzoate de sodium | | 0,20 |
| Carboxyméthyl cellulose | | 1,20 |
| Polysorbate 80 | | 0,60 |
| Microcapsules silicones à diffusion contrôlée diamètre moyen 5 µ contenant : | | 7,00 |
| Huile essentielle de Menthe poivrée | *100,00* | |
| Microcapsules gélatine réticulée de poisson diamètre moyen 40 µ faible épaisseur à rupture mécanique contenant : | | 3,00 |
| Chlorhexidine digluconate | *2,00* | |
| Huile essentielle de Citron | *98,00* | |
| TOTAL | | 100,00 |

Les microcapsules en silicone à diffusion contrôlée sont préparées par polymérisation in situ.

Les microcapsules à rupture mécanique en gélatine faible épaisseur sont préparées par coacervation complexe en présence d'alginate.

Une lingette en forme de doigtier, constituée d'une couche de cellulose d'épaisseur 50 µm, doublée d'un film imperméable de 20 µm d'épaisseur a été imprégnée par pulvérisation de la partie cellulosique avec la composition indiquée dans le tableau ci-dessus, la composition hors microcapsules étant préparée par simple mélange à la température de 45°C ; après refroidissement à 30°C maximum, les microcapsules y sont ajoutées sous faible agitation. Les microcapsules silicone à diffusion contrôlée sont intégrées sous forme d'une épaisse suspension aqueuse à la concentration de 35% de capsules sèches et les microcapsules de gélatine de poisson à rupture mécanique se présentent sous forme de capsules sèches et sont introduites en fin de procédé de mélange.

### Exemple 4 : Nettoyant dentaire 4

| Composant | % dans microcapsule | % dans formule |
|---|---|---|
| Eau purifiée | | 83,70 |
| Carbomer 940 | | 1,80 |
| Xylitol | | 0,80 |
| Bicarbonate de sodium | | 2,00 |
| Benzoate de sodium | | 0,30 |
| Triéthanolamine | | 0,30 |
| Extrait de camomille | | 0,50 |
| Carraghénane | | 0,60 |
| Microcapsules gélatine réticulée à diffusion contrôlée diamètre moyen 2 µ contenant : | | 7,00 |
| Huile essentielle de Menthe poivrée | *80,00* | |
| Huile essentielle d'Orange douce | *20,00* | |
| Microcapsules de polyamide de diamètre moyen 18 µ à rupture mécanique contenant : | | 3,00 |
| Huile essentielle d'Orange douce | *30,00* | |
| Huile essentielle de Citron | *70,00* | |
| TOTAL | | 100,00 |

Les microcapsules en gélatine réticulée à diffusion contrôlée sont préparées par coacervation complexe.

Les microcapsules à rupture mécanique en polyamide sont préparées par polymérisation in situ.

Une lingette en forme de doigtier, constituée d'une couche de cellulose d'épaisseur 40 µm a été imprégnée par pulvérisation de la partie cellulosique avec la composition indiquée dans le tableau ci-dessus, la composition hors microcapsules étant préparée par simple mélange à la température de 45°C ; après refroidissement à 30°C maximum, les microcapsules y sont ajoutées sous faible agitation. Les deux types de microcapsules sont intégrés sous forme d'une épaisse suspension aqueuse (slurry) à la concentration de 40% de capsules sèches.

## Revendications

1. Lingette de nettoyage dentaire comprenant un support textile non-tissé imprégné d'une composition aqueuse d'hygiène dentaire dont au moins une partie des constituants sont encapsulés dans microcapsules déposées à la surface dudit support, laquelle lingette comprenant deux types différents de microcapsules :
(a) des microcapsules à rupture mécanique :
au moins 80% des microcapsules à rupture mécanique doivent pouvoir résister à une pression égaie à 20 g/cm2, et environ 90% de ces microcapsules doivent se rompre pour libérer leur contenu par rupture de leur enveloppe à une pression égale à 500 g/cm2, la membrane de ces microcapsules devant être substantiellement insoluble dans l'eau ou rendue insoluble dans l'eau par un agent réticulant ou précipitant; elle est constituée, par exemple, de gélatine réticulée, de carboxyméthylcellulose réticulée, de polyamide, de polyacrylate réticulé, de silicone, de résine de mélamine à faible taux de formol résiduel, d'alcool polyvinylique réticulé par le borax, de chitosane, ou plus généralement d'un polymère insoluble dans l'eau obtenu par coacervation complexe, ou polymérisation in situ ou interfaciale, et
(b) des microcapsules à diffusion contrôlée :
pouvant résister sans rupture à la pression exercée lors de l'application de la lingette et, dans tous les cas, à des pressions d'au moins 2 000 g/cm2,
et dont le contenu est libéré par perméation à travers l'enveloppe, laquelle présente une porosité contrôlable par son épaisseur et son degré de réticulation, la membrane de ces microcapsules devant être substantiellement insoluble dans l'eau ou rendue insoluble dans l'eau par un agent réticulant ou précipitant ; elle est constituée, par exemple, de gélatine incomplètement réticulée, de carboxyméthyl cellulose incomplètement réticulée, de polyamide, de silicone, ou plus généralement d'un polymère insoluble dans l'eau obtenu par coacervation complexe ou polymérisation in situ ou interfaciale.

2. Lingette de nettoyage dentaire selon la revendication 1, **caractérisée en ce que** ladite lingette se présente sous forme d'un doigt de gant.

3. Lingette de nettoyage dentaire selon une des revendications 1 à 2, **caractérisée en ce que** le support non tissé est choisi parmi la cellulose, la cellulose modifiée chimiquement et les matières absorbantes dérivées de cellulose.

4. Lingette de nettoyage dentaire selon une des revendications 1 à 2, **caractérisée en ce que** l'épaisseur du support non tissé est comprise entre 20 et 1500 µm, et est au moins égale à 2 fois le diamètre moyen des microcapsules déposées à sa surface.

5. Lingette de nettoyage dentaire selon une des revendications 1 à 2, **caractérisée en ce que** le diamètre moyen des microcapsules déposées à sa surface est compris entre 1 et 50 µm.

6. Lingette de nettoyage dentaire selon une des revendications 1 à 2, **caractérisée en ce que** la quantité de microcapsules déposée sur le non tissé est de 5 à 50 g/m2 de surface du support non tissé.

## Patentansprüche

1. Zahnreinigungstuch, umfassend einen textilen Vliesträger, der mit einer wässrigen Zahnpflegezusammensetzung imprägniert ist, von der mindestens ein Teil der Bestandteile in Mikrokapseln eingekapselt ist, die auf der Oberfläche des Trägers abgelagert sind, wobei das Tuch zwei verschiedene Arten von Mikrokapseln umfasst:
(a) mechanisch zerbrechliche Mikrokapseln:
Mindestens 80% der mechanisch zerbrechlichen Mikrokapseln müssen einem Druck von 20 g/cm2 standhalten können, und etwa 90% dieser Mikrokapseln müssen zur Freisetzung ihres Inhalts durch Zerbrechen ihrer Hülle bei einem Druck von 500 g/cm2 zerbrechen, wobei die Membran dieser Mikrokapseln im Wesentlichen wasserunlöslich sein muss oder durch ein Vernetzungs- oder Fällungsmittel wasserunlöslich gemacht werden muss; Sie besteht z. B. aus vernetzter Gelatine, vernetzter Carboxymethylcellulose, Polyamid, vernetztem Polyacrylat, Silikon, Melaminharz mit niedrigem Restformaldehydgehalt, Alkohol mit Borax vernetztem Polyvinyl, Chitosan oder ganz allgemein einem wasserunlöslichen Polymer, das durch komplexe Koazervation oder In-situ- oder Grenzflächenpolymerisation erhalten wird, und
(b) diffusionskontrollierte Mikrokapseln :
die dem bei der Anwendung des Wischtuchs ausgeübten Druck und in jedem Fall Drücken von mindestens 2000 g/cm2 ohne Bruch widerstehen können und deren Inhalt durch Permeation durch die Hülle freigesetzt wird, die eine durch ihre Dicke und ihren Vernetzungsgrad kontrollierbare Porosität aufweist, wobei die Membran dieser Mikrokapseln im Wesentlichen wasserunlöslich sein muss oder durch ein Vernetzungs- oder Fällungsmittel wasserunlöslich gemacht werden muss ; Sie besteht z. B. aus unvollständig vernetzter Gelatine, unvollständig vernetzter Carboxymethylcellulose, Polyamid, Silikon oder ganz allgemein aus einem wasserunlöslichen Polymer, das durch komplexe Koazervation oder In-situ- oder Grenzflächenpolymerisation erhalten wird.

2. Zahnreinigungstuch nach Anspruch 1, **dadurch gekennzeichnet, dass** das Tuch in Form eines Handschuhfingers vorliegt.

3. Zahnreinigungstuch nach einem der Ansprüche 1 bis 2, **dadurch gekennzeichnet, dass** der Vliesträger ausgewählt ist aus Zellulose, chemisch modifizierter Zellulose und absorbierenden Materialien, die von Zellulose abgeleitet sind.

4. Zahnreinigungstuch nach einem der Ansprüche 1 bis 2, **dadurch gekennzeichnet, dass** die Dicke des Vliesträgers zwischen 20 und 1500 µm liegt und mindestens das Zweifache des mittleren Durchmessers der auf seiner Oberfläche abgelagerten Mikrokapseln beträgt.

5. Zahnreinigungstuch nach einem der Ansprüche 1 bis 2, **dadurch gekennzeichnet, dass** der mittlere Durchmesser der auf seiner Oberfläche abgeschiedenen Mikrokapseln zwischen 1 und 50 µm liegt.

6. Zahnreinigungstuch nach einem der Ansprüche 1 bis 2, **dadurch gekennzeichnet, dass** die Menge der auf dem Vliesstoff abgelagerten Mikrokapseln 5 bis 50 g/m2 der Oberfläche des Vliesstoffträgers beträgt.

## Claims

1. A dental cleaning wipe comprising a non-woven textile carrier impregnated with an aqueous dental hygiene composition having at least a portion of its constituents encapsulated in microcapsules deposited on the surface of said carrier, which wipe comprises two different types of microcapsules
(a) mechanically breakable microcapsules :
at least 80% of the mechanically rupturable microcapsules must be able to withstand a pressure equal to 20 g/cm2, and about 90% of these microcapsules must rupture to release their contents by rupture of their envelope at a pressure equal to 500 g/cm2, the membrane of these microcapsules having to be substantially insoluble in water or rendered insoluble in water by a crosslinking or precipitating agent; it consists of, for example, cross-linked gelatin, cross-linked carboxymethyl cellulose, polyamide, cross-linked polyacrylate, silicone, melamine resin with a low residual formaldehyde content, polyvinyl alcohol polyvinyl alcohol crosslinked with borax, chitosan, or more generally a water-insoluble polymer obtained by complex coacervation, or in situ or interfacial polymerization, and
(b) controlled diffusion microcapsules :
capable of withstanding without rupture the pressure exerted during application of the wipe and, in any case, pressures of at least 2000 g/cm2, and whose contents are released by permeation through the envelope, which has a porosity controllable by its thickness and degree of crosslinking, the membrane of these microcapsules having to be substantially insoluble in water or rendered insoluble in water by a crosslinking or precipitating agent; it consists, for example, of incompletely crosslinked gelatin,
incompletely crosslinked carboxymethyl cellulose, polyamide, silicone, or more generally of a water-insoluble polymer obtained by complex coacervation or in situ or interfacial polymerization.

2. Dental cleaning wipe according to claim 1, **characterized in that** said wipe is in the form of a glove finger.

3. The dental cleaning wipe according to any of claims 1 to 2, **characterized in that** the non-woven backing is selected from cellulose, chemically modified cellulose and absorbent materials derived from cellulose.

4. Dental cleaning wipe according to one of claims 1 to 2, **characterized in that** the thickness of the non-woven support is between 20 and 1500 µm, and is at least equal to 2 times the average diameter of the microcapsules deposited on its surface.

5. Dental cleaning wipe according to one of claims 1 to 2, **characterized in that** the average diameter of the microcapsules deposited on its surface is between 1 and 50 µm.

6. Dental cleaning wipe according to one of claims 1 to 2, **characterized in that** the quantity of microcapsules deposited on the non-woven fabric is from 5 to 50 g/m2 of surface of the non-woven support.
